# EUROPEAN PATENT APPLICATION

(11) **EP 2 998 291 A1**
(43) Date of publication of application: **23.03.2016**
(21) Application number: 15182635.1
(22) Date of filing: 27.08.2015
(51) Int. Cl.: C07C 303/40, C07C 311/41

(54) **A NOVEL PROCESS TO PREPARE INTERMEDIATES OF HIV-PROTEASE INHIBITORS THEREOF**

(30) Priority: 16.09.2014 IN MU29582014
(71) Applicant: ZCL Chemicals Ltd., 400 059 Mumbia, Maharashtra (IN)
(72) Inventor: AGARWAL, Nand Lal, 393002 Ankleshwar, Dist: Bharuch, Gujarat (IN); HIRPARA, Hitin Maganbhai, 393002 Ankleshwar, Dist: Bharuch, Gujarat (IN); MISTRI, Pranav Popatlal, 393002 Ankleshwar, Dist: Bharuch, Gujarat (IN); PATEL, Nitin Maganbhai, 393002 Ankleshwar, Dist: Bharuch, Gujarat (IN)
(74) Representative: Gislon, Gabriele

(57) **Abstract**

The present invention relates to an industrially feasible and economically viable process for the preparation of (1S,2R)-3-[[4-aminophenyl)-sulfonyl](2-methylpropyl)amino]-2-hydroxy-1-(phenyl-methyl)propyl] amine of formula I and its salt thereof and optionally converting it to HIV-protease inhibitors like Darunavir, Amprenavir or its prodrug Fosamprenavir.

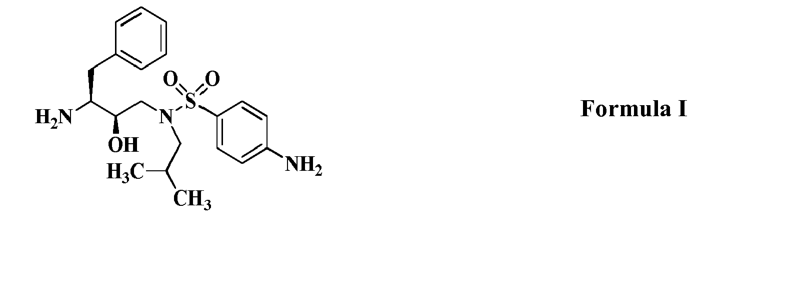

## Description

### FIELD OF THE INVENTION

The present invention relates to a process for the preparation of (1S,2R)-3-[[4-aminophenyl)-sulfonyl](2-methylpropyl)amino]-2-hydroxy-1-(phenylmethyl)propyl]amineof formula I and its salt thereof and optionally converting it to darunavir or its salts or solvates thereof The below cited compound of formula I can be also useful in preparation of HIV-protease inhibitors like Amprenavir or its prodrug Fosamprenavir.

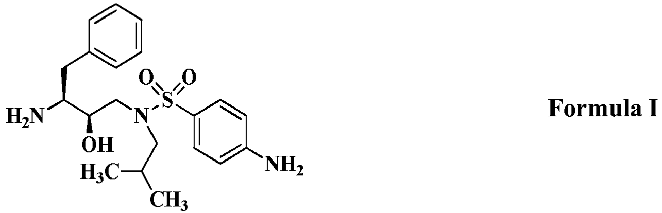

### BACKGROUND OF THE INVENTION

Darunavir is a potent HIV protease inhibitor, chemically known as [(1S,2R)-3-[[(4-aminophenyl)sulfonyl](2-methylpropyl)amino]-2-hydroxy-1-(phenylmethyl) propyl]-carbamic acid (3R,3aS,6aR)hexahydrofuro[2,3-b]furan-3-yl ester as depicted below.

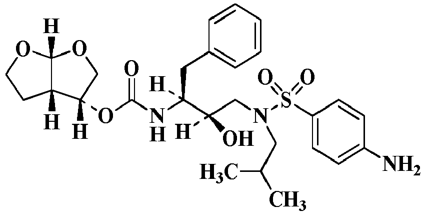

Darunavir is a second-generation protease inhibitor (PIs) used to treat HIV infection. Darunavir is an OARAC recommended treatment option for treatment-naive and treatment-experienced adults and adolescents. Darunavir is marketed under the brand name Prezista® as an oral tablet and oral suspension in the form of monoethanolate solvate.

Darunavir is generically disclosed in US Patent US 5,843,946, specifically disclosed in US patent US 6,248,775. However, in these patents, there is no specific example for preparing Darunavir. US 6,248,775 discloses a process for preparing (1S,2R)-3-[[4-aminophenyl)-sulfonyl](2-methylpropyl)amino]-2-hydroxy-1-(phenyl-methyl) propyl]amine which is referred as diamino alcohol a compound of Formula I in example 18A. The process for the preparation of formula I is depicted in below scheme-1.

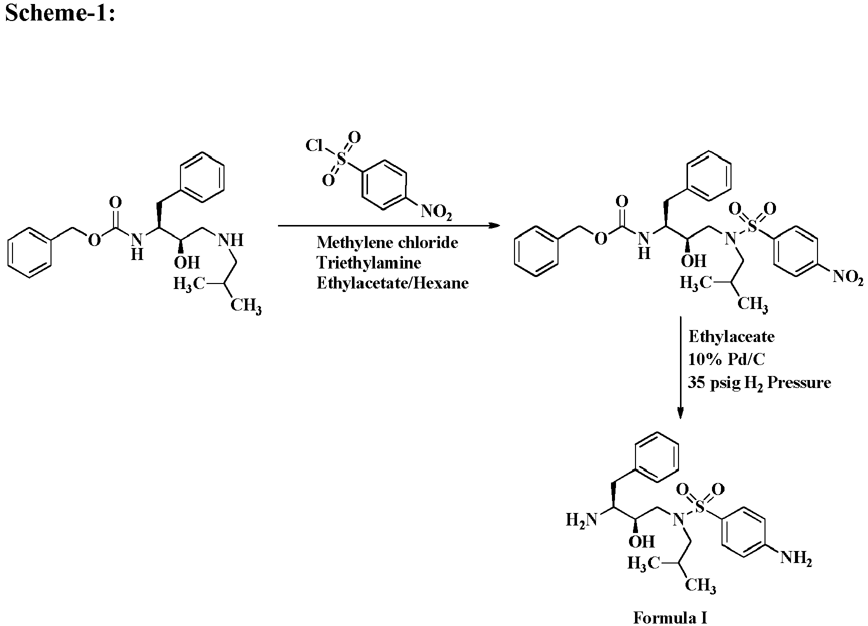

The process is not disclosing purity at any stage.

US 7,772,411 B2 discloses a process to prepare diamino alcohol a compound of Formula I which is as shown in below scheme-2.

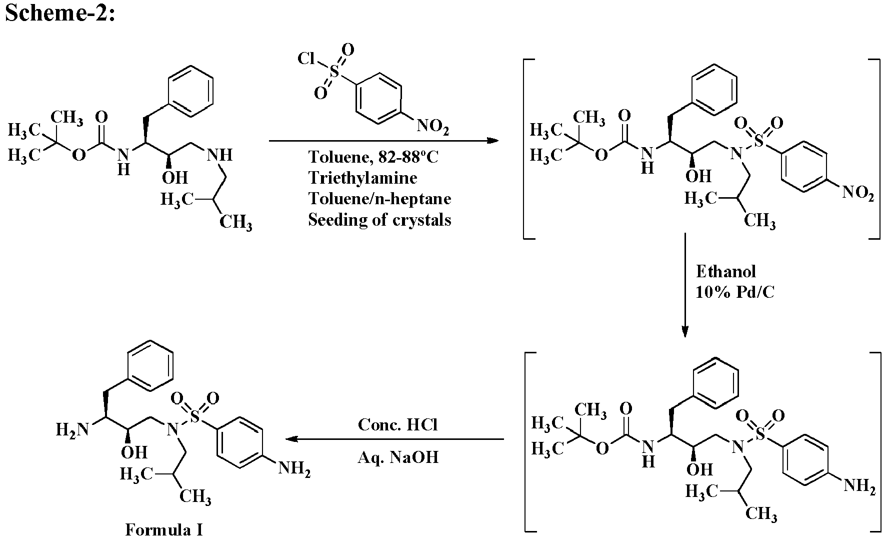

Patent application is silent about the purity of diamino alcohol.It is evidently reported in WO 2013/108105, that when above process is followed, there are strong chances to generate the impurities during reaction. It is said that unwanted reactions observed during reaction which leads the formation of nitro compound as cited below and may be carried forward up to the final product.

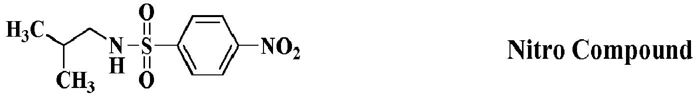

WO 2013/108105 discloses a process to prepare darunavir glycol solvate via diamino alcohol monohydrochloride which is as shown in below scheme-3.

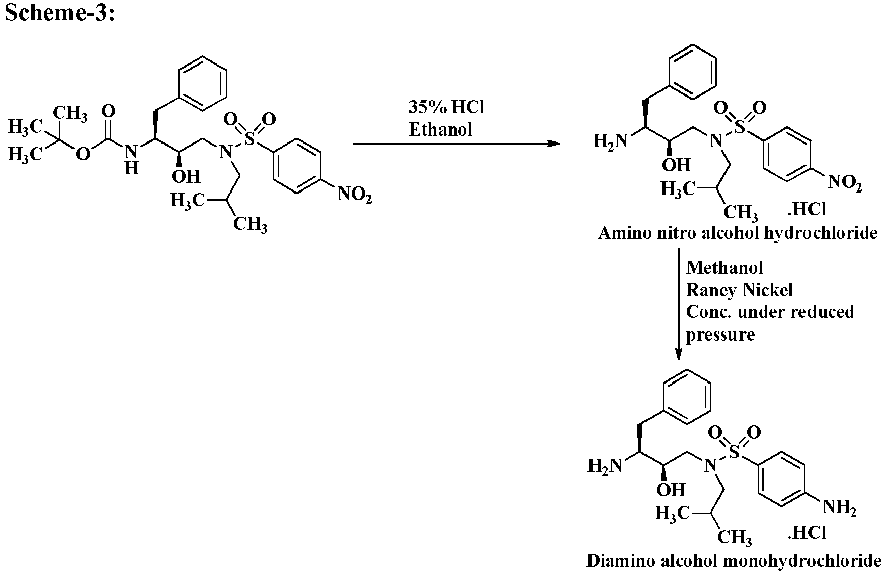

The major disadvantage of the above process is starting material [(1S, 2R)-3-[[(4-Nitrophenylsulfonyl](2-methylpropyl)]amino]-2-hydroxy-1-(phenylmethyl)propyl]carbamic acid tert-butyl ester has to be taken of very pure quality preferably more than 99.8% as patent application is disclosing the purity of diamino alcohol hydrochloride99.9% by HPLC without purification as there may not be quality improvement possible in-between the process, if the quality of starting material is poor.

WO 2013/011485 discloses process for the preparation of diamino alcohol of formula I which is as shown in below scheme-4.

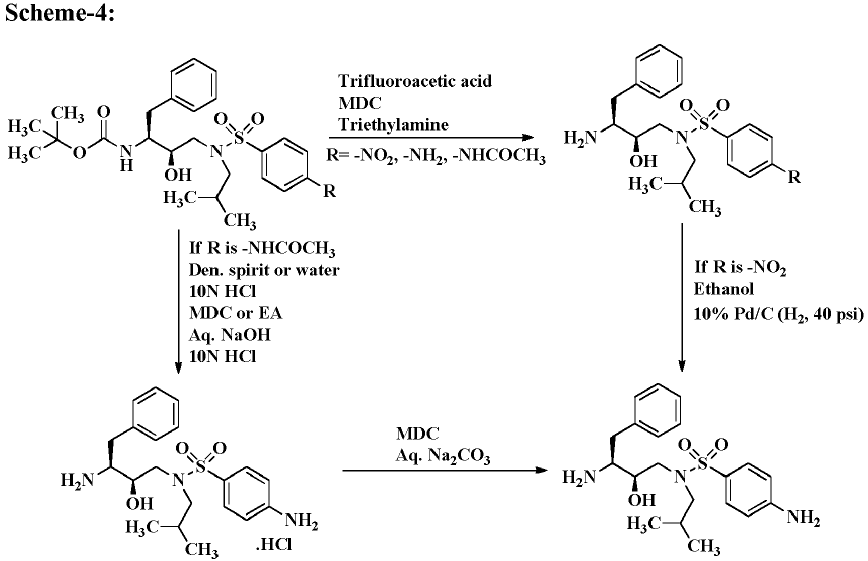

There is no disclosure of purity of diamino alcohol free base and yield reported is low. Hence there may be impurity carried forward upto the final stage of active pharmaceutical ingredient or may be more purifications are required at final stage to achieve regulatory acceptable purity.

Thus, there is strong need to design the new process which is able to ameliorate the disadvantages of the above cited prior art processes. Hence, present invention fulfills the need of the art to provide an improved, industrially applicable and economically viable process for preparation of diamino alcohol having high purity as well as good yieldas cited in scheme-5 via salt formation of formula III which leads the preparation of diamino alcohol in purer form preferably greater than 99.8%, which can be further converted to Darunavir, Amprenavir, its prodrug Fosamprenaviror its pharmaceutically acceptable salts, solvates thereof in high purity as well as good yield.

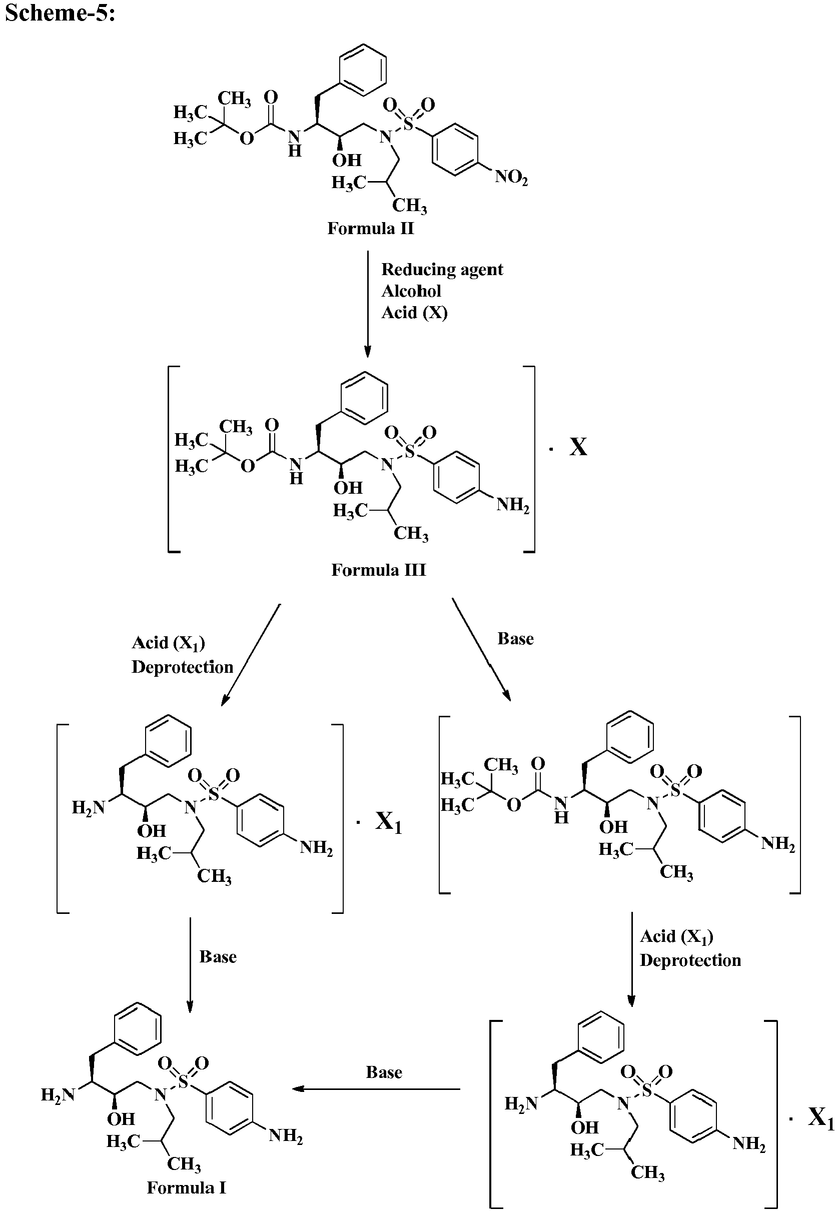

### OBJECTIVE OF THE INVENTION

The principal objective of the present invention is to provide a process for preparation of diamino alcohol of formula Ito overcome or ameliorate one of the disadvantages of the prior art processes.

Another leading objective of the present invention is to provide diamino alcohol of formula I in high yield as well as high quality.

Yet another primary object of the present invention is to provide a novel salts of intermediates and its isolation techniques.

Yet another principleobjective of the present invention is to provide a novel technique of direct acid salt replacement.

Another prime objective of the present invention is to provide an efficient, improved and industrially advantageous process for preparation of diamino alcohol of formula I which is conveniently applicable to industrial scale.

Another key objective of the present invention is to provide a process for the preparation of diamino alcohol of formula I which can be further converted into HIV-protease inhibitors like Darunavir, Amprenavir or Fosamprenavir, its salts or solvates thereof.

### SUMMARY OF THE INVENTION

Accordingly, the present invention provides a process for the preparation of diamino alcohol of formula I, 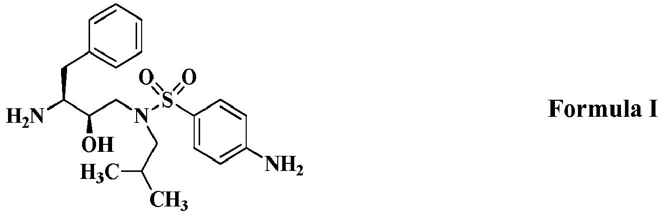 comprises the steps of:
a) reactingcompound of formula II with reducing agent in suitable solvent;
b) treating the reaction mixture with acid to form compound of formula III;
c) optionally isolating compound of formula III;
d) deprotecting compound of formula III;
e) isolating compound of formula I; and
f) optionally converting formula I into HIV-protease inhibitors like

Darunavir, Amprenavir or its prodrug Fosamprenavir.

Accordingly, the present invention provides a process for the preparation of compound of formula III comprises the step of reacting compound of formula II with reducing agent in suitable solvent followed by reacting with organic acid to form compound of formula III.

Accordingly, the present invention provides a process for the preparation of compound of formula I from compound of formula III comprises the steps of:
a) reacting compound of formula III directly with deprotecting agent;
b) treating reaction mixture obtained from step a) with organic or inorganic base to isolate compound of formula I; and
c) optionally converting formula I into HIV-protease inhibitors like Darunavir, Amprenavir or its prodrug Fosamprenavir.

Accordingly, the present invention provides a process for the preparation of compound of formula I from compound of formula III comprises the steps of:
a) reacting compound of formula III with organic or inorganic base to form free base of formula III;
b) treating reaction mixture obtained from step a) with deprotecting agent;
c) treating reaction mixture obtained from step b) with organic or inorganic base to isolate compound of formula I; and
d) optionally converting formula I into HIV-protease inhibitors like Darunavir, Amprenavir or its prodrug Fosamprenavir.

Accordingly, the present invention provides compound of formula III, 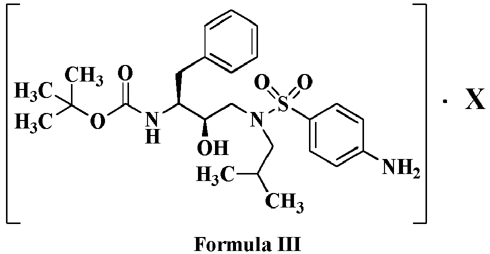 wherein X represents organic acid selected from oxalic acid, malic acid, malonic acid, citric acid, tartaric acid, fumaric acid and the like

### DETAILED DESCRIPTION OF THE INVENTION

All ranges recited herein include the endpoints, including those that recite a range "between" two values. Terms such as "about", "generally" and the like are to be construed as modifying a term or value such that it is not an absolute. Such terms will be defined by the circumstances and the terms that they modify as those terms are understood by those skill in the art. This includes, at very least, the degree of expected experimental error, technique error and instrument error for a given technique used to measure a value.

According to theembodiment of the invention provides an industrially feasible and economically viable process for preparation of diamino alcohol of formula I.

### Stage 1:

Generally the reaction involves reduction and acid addition salt formation in single step in which reaction involves treatment of [(1S,2R)-3-[[(4-nitrophenylsulfonyl](2-methylpropyl)]amino]-2-hydroxy-1-(phenylmethyl)propyl] carbamic acid tert-butyl ester (herein after Boc Nitro) of formula II with reducing agent in suitable solvent to provide free base of formula III which is then treated with organic acid (X) in suitable solvent to form acid addition salt of formula III.

Accordingly the reduction reaction involves reducing agent include palladium on carbon, raney Nickel, palladium hydroxide, platinum on carbon, platinum oxide, hydrazine hydrate and the like preferably raney Nickel and palladium on carbon. The suitable solvent can be selected from water, alcohols such as methanol, ethanol, isopropanol, butanols and the like, ester such as ethyl acetate, amides such as dimethylformamide, acetic acid, dichloromethane, toluene, xylene, benzene, pentane, hexane, heptane, petroether, 1,4-thioxane, diethyl ether, diisopropyl ether, tetrahydrofuran, 1,4-dioxane, 1,2-dimethoxyethane, dimethyl sulphoxide or mixtures thereof, preferably alcohol. The reaction can be carried out at hydrogen pressure 5 to 6 kg/cm² and atambient to reflux temperature, preferably between 20-40°C, more preferably 25-35°C. The hydrogen gas purging preferably can be continued up to the absence of the starting material. The completion of the reaction confirmed by thin layer chromatography or high performance liquid chromatography. According to a preferred embodiment, the reduction step is performed using reducing agent palladium on charcoal or raney Nickel in methanol or isopropanol as reaction media. After removal of pressure, the reaction mixture can be filtered and washed with suitable solvent. The solvent may be distilled out completely or partially from the reaction mixture and cooled the mixture. Again the suitable solventcan be added to proceed further for acid salt formation. The suitable solvent can be selected from water, alcohols such as methanol, ethanol, isopropanol, butanols and the like, ester such as ethyl acetate, amides such as dimethylformamide, acetic acid, dichloromethane, toluene, xylene, benzene, pentane, hexane, heptane, petroether, 1,4-thioxane, diethyl ether, diisopropyl ether, tetrahydrofuran, 1,4-dioxane, 1,2-dimethoxyethane, dimethyl sulphoxide or mixtures thereof, preferably toluene or xylene. The organic acid (X) can be organic acid such as oxalic acid, malic acid, malonic acid, citric acid, tartaric acid, fumaric acid and the like. The reaction temperature can be from ambient to reflux temperature, preferably between 25-40°C.The completion of the reaction may be confirmed by thin layer chromatography. The compound of formula III can be isolated from the reaction mixture by suitable techniques such as filtration or centrifugation and the like. Dried the compound by the conventional techniques know in the art. The process resulted in desired compound in high purity and good yield.Alternatively the compound of formula III may be proceed *in-situ* for further reaction.

### Stage 2:

Generally the reaction involves deprotection of compound of formula III in which amino group is protected by *tert*-butoxy carbonyl group to convert into compound of formula I (or diamino alcohol or diamine) by treating with acid (X₁) for the deprotection purpose. In another way, the reaction can be defined by a salt replacement or salt exchange reaction. The process involves replacement of one acid salt (X) by another acid salt (X₁) in reaction media. Alternatively, the acid salt of formula III can be converted to free base by reacting with inorganic base and then treated with the acid for the deprotection of protected amino group. Acid (X₁) employed for the removal of amino protecting group include inorganic acid such as hydrochloric acid, hydrobromic acid, nitric acid, sulfuric acid and phosphoric acid or mixtures thereof, organic acid such as acetic acid, trifluoroacetic acid, methanesulphonic acid and *p*-toluene sulphonic acid and the like or mixtures thereof. According to preferred embodiment, the deprotection step is performed using hydrochloric acid. The solvent employed during the deprotection is not particularly limited provided that it has no adverse effect on the reaction and dissolves the starting materials to at least some extent. The suitable solvent may employed such aliphatic hydrocarbons such as alcohols such as methanol, ethanol, propanol, isopropanol and butanol; hexane, heptane and petroleum ether; aromatic hydrocarbons such as benzene, toluene, xylene and mesitylene; halogenated hydrocarbons such as methylene chloride, chloroform, carbon tetrachloride and dichloroethane; ethers such as diethyl ether, tetrahydrofuran, 1,4-dioxane and 1,2-dimethoxyethane; esters such as methyl acetate, ethyl acetate, methyl propionate and ethyl propionate; nitrites such as acetonitrile; amides such as N,N-dimethylformamide and N,N-dimethylacetamide; sulfoxides such as dimethyl sulfoxide and mixtures thereof. Alcohols are preferred solvents. The reaction mixture heated at ambient to reflux temperature, preferably 20-40°C, more preferably 25-35°C.The reaction mixture can be maintained up to the absence of starting material. The completion of the reaction can be confirmed by thin layer chromatography or high performance liquid chromatography. The acid salt of compound of formula I can be isolated from the reaction mixture and dried by the suitable techniques know in the art.

Further the acid salt of compound of formula I can be used directly for the preparation of HIV-protease inhibitors like Darunavir, Amprenavir or its prodrug Fosamprenavir.

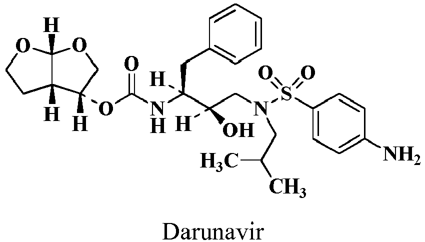

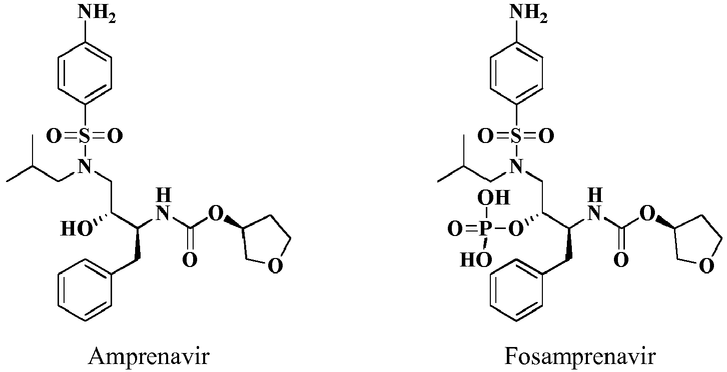

Alternatively, the obtained acid salt of formula I can be converted into the free base form of formula I by treating with inorganic base such as sodium hydroxide, sodium carbonate, potassium hydroxide, lithium hydroxide, ammonia, hydrazine, calcium hydroxide, any of the bases listed above, and mixtures thereof. Compound of formula I in the form of free base can be isolated from the reaction mixture by suitable techniques such as filtration or centrifugation and the like. Dried the compound by the conventional techniques know in the art. The process resulted in desired compound of formula I in high purity, preferably greater than 99.8%, more preferably greater than 99.9% and good yield.

Further the free base of formula I can be used for the preparation of HIV-protease inhibitors like Darunavir, Amprenavir or its prodrug Fosamprenavir.

The invention is further defined by reference to the following examples describing in detail by the preparation of the compounds of the invention. It will be apparent to those skilled in the art that many modifications, both to materials and methods, may be practiced without departing from the scope of the invention.

### EXAMPLES:

### Part A: [Boc-Nitro → dry oxalate salt (Formula III) → Diamino alcohol (Formula I)]

### Example-1: Preparation of 1-Benzyl-2-hydroxy-3-[isobutyl-(4-amino-benzene sulfonyl) amino] propyl)carbamic acid tert-butyl ester oxalatefrom BOC Nitro usingRaney Nickel and isopropyl alcohol

BOC Nitro (100gm) (0.1917mol) andRaney Nickel (5gm) (5%w/w) catalyst was added in isopropyl alcohol (1000ml) at 25-35°C then maintained 5 to 6 kg/cm² pressure of hydrogen gas up to absence of starting material and was checked by TLC. Released the pressure, filtered the reaction mass and washed with isopropyl alcohol (150ml). Distilled out isopropyl alcohol (1050ml) under reduced pressure. Cooled the mass at 25-30°Cand toluene (1000ml) was added. Stirred the reaction mixture and oxalic acid dihydrate (26.5gm) (0.21mole) was added at 25-30°C. The reaction mixture was stirred at 25-30°C for 1hr, cooled to 0-5°C and further stirred for 2hrs. Filtered the precipitated mass and washed the solid with mixture of toluene and isopropyl alcohol (10:1) (100ml). Dried the obtained wet cake at 45-50°C under vacuum to get 100gm of the title compound.
*Yield: 1.00w*/*w, % Yield: 90%, HPLC purity: >98.9%*

### Example-2: Preparation of 1-Benzyl-2-hydroxy-3-[isobutyl-(4-amino-benzene sulfonyl) amino] propyl)carbamic acid tert-butyl ester oxalatefrom BOC Nitro using Raney Nickel and Methanol

BOC Nitro (100gm) (0.1917mol) and Raney Nickel (5gm) (5%w/w) catalyst was added in methanol (500ml) at 25-35°C then maintained 5 to 6 kg/cm² pressure of hydrogen gas up to absence of starting material and was checked by TLC. Released the pressure, filtered the reaction mass and washed with methanol (150ml). Distilled out methanol completely under reduced pressure. Cooled the mass and mixture of isopropyl alcohol (100ml) and toluene (1000ml) was added. Stirred the reaction mixture and oxalic acid dihydrate (26.5gm) (0.21mole) was added at 25-30°C. The reaction mixture was stirred at 25-30°C for 1hr, cooled to 0-5°C and further stirred for 2hrs. Filtered the precipitated mass and washed the solid with mixture of toluene and isopropyl alcohol (10:1) (100ml). Dried the obtained wet cake at 45-50°C under vacuum to get 100gm of the title compound.
*Yield: 1.00w*/*w, % Yield: 90%, HPLC purity: >98.9%*

### Example-3: Preparation of 1-Benzyl-2-hydroxy-3-[isobutyl-(4-amino-benzene sulfonyl) amino] propyl)carbamic acid tert-butyl ester oxalatefrom BOC Nitro using Palladium on carbon and Isopropyl alcohol

BOC Nitro (100gm) (0.1917mol) and Palladium on carbon (5gm)(5%w/w) (50% wet) catalyst was added in isopropyl alcohol (500ml) at 25-35°C then maintained 5 to 6 kg/cm² pressure of hydrogen gas up to absence of starting material and was checked by TLC. Released the pressure, filtered the reaction mass and washed with isopropyl alcohol (150ml). Distilled out isopropyl alcohol (550ml) under reduced pressure. Cooled the mass and toluene (1000ml) was added. Stirred and oxalic acid dihydrate (26.5gm) (0.21mole) was added at 25-30°C. Stirred at 25-30°C for 1hr, cooled to 0-5°C and further stirred for 2hrs. Filtered the precipitated mass and washed the obtained solid with mixture of toluene and isopropyl alcohol (10:1) (100ml). Dried the obtained wet cake at 45-50°C under vacuum to get 100gm of the title compound.
*Yield: 1.00w*/*w, % Yield: 90%, HPLC purity: >98.9%*

### Example-4: Preparation of 1-Benzyl-2-hydroxy-3-[isobutyl-(4-amino-benzene sulfonyl) amino] propyl)carbamic acid tert-butyl ester oxalate from BOC Nitro using Palladium on carbon and Methanol

BOC Nitro (100gm) (0.1917mol) andPalladium on carbon (5gm)(5%w/w) (50% wet) catalyst was added in methanol (500ml) at 25-35°C then maintained 5 to 6 kg/cm² pressure of hydrogen gas up to absence of starting material and was checked by TLC. Released the pressure, filtered the reaction mass and washed with methanol (150ml). Distilled out methanol completely under reduced pressure. Mixture of isopropyl alcohol (100ml) and toluene (1000ml) was added into the reaction mixture. Stirred and oxalic acid dihydrate (26.5gm) (0.21mole) was added at 25-30°C. Stirred at 25-30°C for 1hr, cooled to 0-5°C and further stirred for 2hrs. Filtered the precipitated mass and washed the obtained solid with mixture of toluene and isopropyl alcohol (10:1) (100ml). Dried the obtained wet cake at 45-50°C under vacuum to get 100gm of the title compound.
*Yield: 1.00w*/*w, % Yield: 90%, HPLC purity:* 98.88%.

### Example-5: Preparation of 4-Amino-N-((2R,3S)-3-amino-2-hydroxy-4-phenyl butyl)-N-isobutyl) benzene sulfonamidefrom dry oxalate salt

1-Benzyl-2-hydroxy-3-[isobutyl-(4-amino-benzenesulfonyl) amino] propyl) carbamic acid tert-butyl ester oxalate (100gm) (0.172mol) was added in methanol (300ml) followed by addition of concentrated hydrochloric acid (170.7gm) (1.40mol) at 25-35°C and maintained it up to absence of starting material and was checked by TLC. Distilled out the methanol under reduced pressure below 45°C. The reaction mixture was cooled to 25-35°C, purified water (1050ml) was added and mixed well. Quenched the acidic mass in a sodium hydroxide solution [Prepared a solution of sodium hydroxide (56.12gm)(1.40mol) by dissolved in purified water (825ml)] at 5-10°C mass temperature. Stirred at 5-10°C and filtered the precipitated mass. The obtained solid was washed with purified water (300ml) and suck dried. Dried the obtained wet cake 50-60°C to get 60gm of the title compound.
*Yield: 0.60w*/*w, % Yield: 89.6%, HPLC purity: 99.5%*

### Part B: [Boc-Nitro → Wet oxalate salt (Formula III)→Diamino alcohol (Formula I)]

### Example-6: Preparation of 1-Benzyl-2-hydroxy-3-[isobutyl-(4-amino-benzene sulfonyl) amino] propyl)carbamic acid tert-butyl ester oxalatefrom BOC Nitro using Raney Nickel and methanol

BOC Nitro (100gm) (0.1917mol) andRaney Nickel (5gm)(5%w/w) catalyst was added in methanol (500ml) at 25-35°C then maintained 5 to 6 kg/cm² pressure of hydrogen gas up to absence of starting material and was checked by TLC. Released the pressure, filtered the reaction mass and washed with methanol (150ml). Distilled out methanol completely under reduced pressure. Mixture of isopropyl alcohol (100ml) and toluene (1000ml) was added into mass. Stirred and oxalic acid dihydrate (26.5gm) (0.21mole) was added at 25-30°C. Stirred at 25-30°C for 1hr, cooled to 0-5°C and further stirred for 2hrs. Filtered the precipitated mass and washed the obtained solid with mixture of toluene and isopropyl alcohol (10:1) (100ml) to get 210gm wet cake of the title compound. *Loss on drying: 52.38%; HPLC purity: 98.63%.*

### Example-7: Preparation of 4-Amino-N-((2R,3S)-3-amino-2-hydroxy-4-phenyl butyl)-N-isobutyl) benzene sulfonamide[Wet oxalate salt → Free base →Diamino alcohol via hydrolysis]

1-Benzyl-2-hydroxy-3-[isobutyl-(4-amino-benzenesulfonyl) amino] propyl) carbamic acid tert-butyl ester oxalate (210gm having LOD: 52.38%) (0.172mol) was added in mixture of toluene (600ml) and purified water (700ml). Sodium hydroxide solution (10%) at 25-30°C was added into the reaction mixture and stirred at 25-30°C for 30minutes. Separated the organic layer and purified water (1250ml) as added into it followed by the addition of concentrated hydrochloric acid (170.7gm) at 25-30°C. Stirred the mass at 30-35°C up to absence of starting material about 20 hours and was checked by TLC. Separated the acidic aqueous layer and quenched in the sodium hydroxide solution [Prepared by dissolving sodium hydroxide (63.48gm) in purified water (1037ml)] at 5-10°C. Stirred at 5-10°C and filtered the precipitated mass. The obtained solid was washed with purified water (300ml) and suck dried. Dried the obtained wet cake at 50-60°C to get 53gm of the title compound.
*Yield: 0.53w*/*w, % Yield: 79.10%, HPLC purity: 99.9%*

### Example-8: Preparation of 4-Amino-N-((2R,3S)-3-amino-2-hydroxy-4-phenyl butyl)-N-isobutyl) benzene sulfonamide [Wet oxalate salt →Diamino alcohol via hydrolysis as direct acid salt replacement]

1-Benzyl-2-hydroxy-3-[isobutyl-(4-amino-benzenesulfonyl) amino] propyl) carbamic acid tert-butyl ester oxalate (210gm having LOD: 52.38%) (0.172mol) was added in methanol (300ml) followed by addition of concentrated hydrochloric acid (170.7gm) (1.40mol) at 25-35°C. Maintained it up to absence of starting material and was checked by TLC. Distilled out the methanol under reduced pressure below 45°C. Cool to 25-35°C and purified water (1050ml) was added and mixed well. Quenched the acidic mass in a sodium hydroxide solution [Prepared a solution of sodium hydroxide (56.12gm) (1.40mol) by dissolved in purified water (825ml)] at 5-10°C mass temperature. Stirred at 5-10°C and filtered the precipitated mass. The obtained solid was washed with purified water (300ml) and suck dried. Dried the obtained wet cake at 50-60°C to get 60gm of the title compound.
*Yield: 0.60w*/*w, % Yield: 89.6%, HPLC purity: 99.5%.*

### Part C: [In-situ process for the preparation of Diamino alcohol (Formula I)]

### Example-9:In-situ process for preparation of 4-Amino-N-((2R, 3S)-3-amino-2-hydroxy-4-phenylbutyl)-N-isobutyl) benzene sulfonamide [From BOC Nitro using Raney Nickel and Methanol → Wet oxalate salt → Free base → diamino alcohol via hydrolysis]

BOC Nitro (100gm) (0.1917mol) and Raney Nickel (5gm) (5%w/w)catalyst was added in methanol (500ml) at 25-35°C then taken 5 to 6 kg/cm² pressure of hydrogen gas in autoclave and maintained at 25-35°C up to absence of starting material was checked by TLC. Released the pressure, filtered the reaction mass and washed with methanol (150ml). Distilled out methanol completely under reduced pressure. Mixture of isopropyl alcohol (100ml) and toluene (1000ml) was added into mass. Stirred and oxalic acid dihydrate (26.5gm) (0.21mole) was added at 25-30°C. Stirred at 25-30°C for 1hr, cooled to 0-5°C and further stirred for 2hrs. Filtered the precipitated mass and washed the obtained solid with mixture of toluene and isopropyl alcohol (10:1) (100ml). The obtained wet cake of 1-Benzyl-2-hydroxy-3-[isobutyl-(4-amino-benzenesulfonyl) amino] propyl) carbamic acid tert-butyl ester oxalate (210gm having LOD: 52.38%) (0.172mol) was added in mixture of toluene (600ml) and purified water (700ml). Sodium hydroxide solution (10%) at 25-30°C was added till the pH 12-13 and stirred at 25-30°C for 30minutes. Separated the organic layer and purified water (1000ml) was added into it followed by the addition of concentrated hydrochloric acid (170.7gm) at 25-30°C. Stirred the mass at 30-35°C up to absence of starting material was checked by TLC. Separated the acidic aqueous layer and quenched in sodium hydroxide solution [Prepared by dissolving sodium hydroxide (63.48gm) in purified water (832ml)] at 5-10°C temperature. Stirred at 5-10°C and filtered the precipitated mass. The obtained solid was washed with purified water (300ml) and suck dried. Dried the obtained wet cake at 50-60°C to get 53gm of the title compound.
*Yield: 0.53w*/*w, % Yield: 70.66%, HPLC purity: 99.87%.*

### Example-10: In-situ process for preparation of 4-Amino-N-((2R, 3S)-3-amino-2-hydroxy-4-phenylbutyl)-N-isobutyl) benzene sulfonamide [BOC Nitro using Raney Nickel and Methanol → Wet oxalate salt → Diamino alcohol via hydrolysis as direct acid salt replacement]

BOC Nitro (100gm) (0.1917mol) and Raney Nickel (5gm) (5%w/w)catalyst was added in methanol (500ml) at 25-35°C temperature then taken 5 to 6 kg/cm2 pressure of hydrogen gas in autoclave and maintained at 25-35°C up to absence of starting material was checked by TLC. Released the pressure, filtered the reaction mass and washed with methanol (150ml). Distilled out methanol completely under reduced pressure. Mixture of isopropyl alcohol (100ml) and toluene (1000ml) was added into mass. Stirred and oxalic acid dihydrate (26.5gm) (0.21mole) was added at 25-30°C. Stirred at 25-30°C for 1hr, cooled to 0-5°C and further stirred for 2hrs. Filtered the precipitated mass and washed the obtained solid with mixture of toluene and isopropyl alcohol (10:1) (100ml). The obtained wet cake of 1-Benzyl-2-hydroxy-3-[isobutyl-(4-amino-benzenesulfonyl) amino] propyl) carbamic acid tert-butyl ester oxalate (210gm having LOD: 52.38%) (0.172mol) was added in methanol (300ml) followed by addition of concentrated hydrochloric acid (170.7gm) (1.40mol) at 25-35°C and maintained it up to absence of starting material was checked by TLC. Distilled out the methanol under reduced pressure below 45°C. Cooled to 25-35°C and purified water (1050ml) was added and mixed well. Quenched the acidic mass in a sodium hydroxide solution [Prepared a solution of sodium hydroxide (56.12gm) (1.40mol) by dissolved in purified water (825ml)] at 5-10°C mass temperature]. Stirred at 5-10°C and filtered the precipitated mass. The obtained solid was washed with purified water (300ml) and suck dried. Dried the obtained wet cake at 50-60°C to get 60gm of the title compound.
*Yield: 0.60w*/*w, % Yield: 89.6%, HPLC purity: 99.5%.*

## Claims

1. A process for the preparation of diamino alcohol of formula I having purity greater than 99.8%, 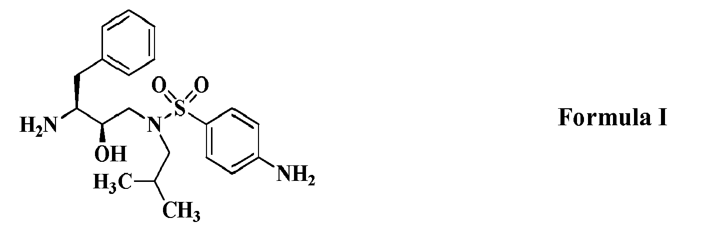 comprising the steps of:
a) reacting compound of formula II with reducing agent in suitable solvent; 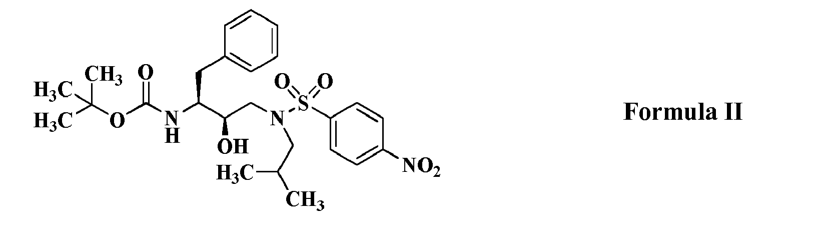
b) treating the reaction mixture with acid to form compound of formula III; 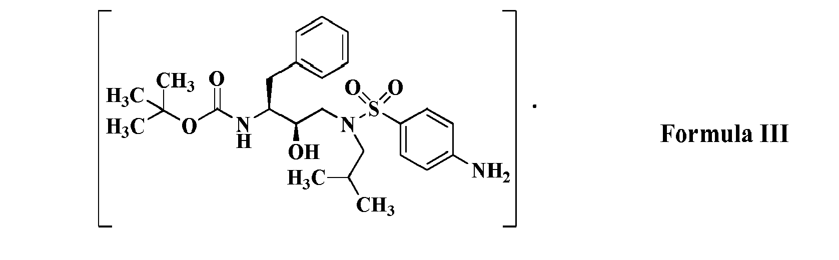
c) optionally isolating compound of formula III;
d) deprotecting compound of formula III;
e) isolating compound of formula I; and
f) optionally converting formula I into HIV-protease inhibitors like Darunavir, Amprenavir or its prodrug Fosamprenavir.

2. The process according to claim 1, wherein
in step a) reducing agent is selected from palladium on carbon, raney Nickel, palladium hydroxide, platinum on carbon, platinum oxide, hydrazine hydrate and the like;
in step a) suitable solvent is selected from water, alcohols such as methanol, ethanol, isopropanol, butanols and the like, ester such as ethyl acetate, amides such as dimethylformamide, acetic acid, dichloromethane, toluene, xylene, benzene, pentane, hexane, heptane, petroether, 1,4-thioxane, diethyl ether, diisopropyl ether, tetrahydrofuran, 1,4-dioxane, 1,2-dimethoxyethane, dimethyl sulphoxide or mixtures thereof;
in step b) acid is selected from oxalic acid, malic acid, malonic acid, citric acid, tartaric acid, fumaric acid and the like;
in step d) deprotecting agent is selected from inorganic acid such as hydrochloric acid, hydrobromic acid, nitric acid, sulfuric acid and phosphoric acid or mixtures thereof, organic acid such as acetic acid, trifluoroacetic acid, methanesulphonic acid and *p*-toluene sulphonic acid and the like or mixtures thereof;
in step d) compound of formula III undergoes deprotection by direct replacement of acid salt with acid or formula III is first converted into the free base form by treating with inorganic or organic base and then undergoes for the deprotection; and
in step e) isolation of formula I is in the form of acid salt or in the form of free base.

3. A process for the preparation of compound of formula III comprising the step of reacting compound of formula II with reducing agent in suitable solvent followed by reacting with organic acid to form compound of formula III.

4. The process according to claim 3, wherein reducing agent is selected from palladium on carbon, raney Nickel, palladium hydroxide, platinum on carbon, platinum oxide, hydrazine hydrate and the like; suitable solvent is selected from water, alcohols such as methanol, ethanol, isopropanol, butanols and the like, ester such as ethyl acetate, amides such as dimethylformamide, acetic acid, dichloromethane, toluene, xylene, benzene, pentane, hexane, heptane, petroether, 1,4-thioxane, diethyl ether, diisopropyl ether, tetrahydrofuran, 1,4-dioxane, 1,2-dimethoxyethane, dimethyl sulphoxide or mixtures thereof; organic acid is selected from oxalic acid, malic acid, malonic acid, citric acid, tartaric acid, fumaric acid and the like.

5. A process for the preparation of compound of formula I having purity greater than 99.8% from compound of formula III comprises the steps of:
a) reacting compound of formula III directly with deprotecting agent;
b) treating reaction mixture obtained from step a) with organic or inorganic base to isolate compound of formula I; and
c) optionally converting formula I into HIV-protease inhibitors like Darunavir, Amprenavir or its prodrug Fosamprenavir.

6. The process according to claim 5, wherein
in step a) deprotecting agent is selected from inorganic acid such as hydrochloric acid, hydrobromic acid, nitric acid, sulfuric acid and phosphoric acid or mixtures thereof, organic acid such as acetic acid, trifluoroacetic acid, methanesulphonic acid and *p*-toluene sulphonic acid and the like or mixtures thereof;
in step a) compound of formula III in the form of acid salt undergoes deprotection by treating with acid via direct replacement of acid without converting compound of formula III in free base form;
in step b) inorganic or organic base is selected from sodium hydroxide, sodium carbonate, potassium hydroxide, lithium hydroxide, ammonia, hydrazine, calcium hydroxide, methylamine, ethylamine, aniline, ethylenediamine, triethylamine, tetraethyl ammonium hydroxide, diisopropylethyl amine, ammonium hydroxide, sodium methoxide, potassium methoxide, any of the bases listed above, and mixtures thereof.

7. A process for the preparation of compound of formula I having purity greater than 99.8% from compound of formula III comprises the steps of:
a) reacting compound of formula III with organic or inorganic base to form free base of formula III;
b) treating reaction mixture obtained from step a) with deprotecting agent;
c) treating reaction mixture obtained from step b) with organic or inorganic base to isolate compound of formula I; and
d) optionally converting formula I into HIV-protease inhibitors like Darunavir, Amprenavir or its prodrug Fosamprenavir.

8. The process according to claim 7, wherein
in step a) and step c) inorganic base is selected from sodium hydroxide, sodium carbonate, potassium hydroxide, lithium hydroxide, ammonia, hydrazine, calcium hydroxide and ammonium hydroxide, any of the bases listed above, and mixtures thereof;
in step b) deprotecting agent is selected from inorganic acid such as hydrochloric acid, hydrobromic acid, nitric acid, sulfuric acid and phosphoric acid or mixtures thereof, organic acid such as acetic acid, trifluoroacetic acid, methanesulphonic acid and *p*-toluene sulphonic acid and the like or mixtures thereof.

9. The process according any of above proceeding claim, wherein compound of formula I having purity greater than 99.8% is converted to HIV protease inhibitor by any known method in the art.

10. Compound of formula III, 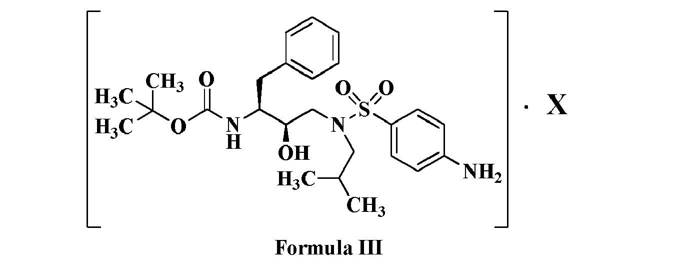 wherein X represents organic acid selected from oxalic acid, malic acid, malonic acid, citric acid, tartaric acid, fumaric acid.
